# EUROPEAN PATENT APPLICATION

(11) **EP 3 682 748 A1**
(43) Date of publication of application: **22.07.2020**
(21) Application number: 19152027.9
(22) Date of filing: 16.01.2019
(51) Int. Cl.: A23L 33/195, A23C 9/12, C12N 9/38

(54) **PAENIBACILLUS WYNNII BETA-GALACTOSIDASE FOR THE PRODUCTION OF LACTOSE-DEPLETED DAIRY PRODUCTS**

(71) Applicant: Universität Hohenheim, 70593 Stuttgart (DE)
(72) Inventor: FISCHER, Lutz, 73274 Notzingen (DE); LUTZ-WAHL, Sabine, 73230 Kirchheim unter Teck (DE)
(74) Representative: Müller-Boré & Partner Patentanwälte PartG mbB

(57) **Abstract**

The present invention relates to uses of a specific enzyme having beta-galactosidase activity from Paenibacillus wynnii in the production of lactose-depleted or lactose-free dairy products, as well as respective methods for the production of a lactose-depleted or lactose-free dairy product.

## Description

The present invention relates to uses of a specific enzyme having beta-galactosidase activity in the production of lactose-depleted or lactose-free dairy products, as well as respective methods for the production of a lactose-depleted or lactose-free dairy product.

For the enzymatic hydrolysis of milk and whey products, numerous processes with soluble and immobilized enzyme preparations have been described. If a soluble enzyme preparation is used, lactose is usually hydrolysed batchwise after pasteurization. Hydrolysis overnight at low temperatures is preferred, as this is more practicable than hydrolysis for e. g. 4 hours at 37°C, where the milk must be processed immediately after hydrolysis.

In the production of UHT (ultra-high temperature processed) milk, enzymes may be added aseptically after heat treatment. However, the requirements for enzyme purity are higher and the enzyme is present in active form in the end product.

Since the enzyme costs are critical for these processes, various immobilization methods with beta-galactosidases have been investigated. In Italy, lactose hydrolysis in milk has long been described with immobilized beta-galactosidase.

In addition to enzymatic reduction of the lactose concentration in milk and whey products, there is also the possibility of selective chromatographic separation of lactose. The world market leader in lactose-free dairy products has been using a combined process, using both enzymatic hydrolysis and selective chromatographic separation of lactose from milk to produce lactose-free milk.

In earlier studies, a new industry-relevant galactosidase has already been found in metagenome studies. A metagenome library of 1.3 million clones was screened for new beta-galactosidases using a three-stage activity-based screening. Six metagenome beta-galactosidases showed improved lactose hydrolysis in milk compared to the commercial enzyme preparation GODO-YNL2 from *Kluyveromyces lactis.* The best candidate beta-galactosidase M1 showed a higher affinity to the substrate lactose and a lower product inhibition to the resulting galactose than the commercial enzyme preparation.

Beta-galactosidases from *Paenibacillus thiaminolyticus* and *Paenibacillus barengoltzii* have already been described in this respect. The beta-galactosidase from *P. thiaminolyticus* had a very low affinity to the substrate lactose. The *K*_{M,lactose} value was 206 ± 5 mM. However, the determination was carried out at pH 6 and at a temperature of 37 °C. For the beta-galactosidase PbGal2A from *P*. *barengoltzii,* the *K*_{M*,*lactose} value of 43.27 ± 1.84 m*M* was significantly higher.

Thus, although a considerable number of studies have been published investigating beta-galactosidases from different sources for lactose hydrolysis, only a small number of enzyme preparations, mainly obtained from the yeasts *K*. *lactis* and *K. fragilis,* are applied for industrial applications. However, the industrially used enzymes possess serious disadvantages.

The beta-galactosidases utilised in the food industry are significantly inhibited by the hydrolysis-generated product D-galactose whereas the substrate affinity to the substrate lactose is not favourable (*i.e.,* the *K*_{M}-value for lactose is too high (>10 m*M*)). As a result, the enzymatic hydrolysis of lactose in food requires a correspondingly large amount of enzymes (cost factor) and the time required for effective lactose hydrolysis is high (time factor). The latter entails a high risk of contamination of the process.

Therefore, the technical problem underlying the present invention is the provision of beta-galactosidases for use in the production of lactose-depleted and lactose-free dairy products which are characterized by low product inhibition, high substrate affinity and high enzyme activity.

The solution to the above technical problem is achieved by the embodiments characterized in the claims.

In particular, in a first aspect, the present invention relates to the use of an enzyme having beta-galactosidase activity in the production of lactose-depleted or lactose-free dairy products, wherein said enzyme comprises
(a) the amino acid sequence of SEQ ID NO: 1; or
(b) an amino acid sequence having at least 70% sequence identity to SEQ ID NO: 1 and having beta-galactosidase activity.

As used herein, the term "enzyme having beta-galactosidase activity" relates to a glycoside hydrolase enzyme that catalyses the hydrolysis of terminal nonreducing beta-D-galactose residues in beta-D-galactosides through the breaking of a glycosidic bond.

The enzyme having beta-galactosidase enzyme activity used in the present invention is either (i) the *P. wynnii* beta-galactosidase having the amino acid sequence of SEQ ID NO: 1, the advantageous use of which in the production of lactose-depleted or lactose-free dairy products has been discovered in the present invention, or (ii), in the case of an enzyme having at least 70% sequence identity to SEQ ID NO: 1 and having beta-galactosidase activity, a respective beta-galactosidase derived therefrom.

The term "lactose-depleted dairy product" as used herein relates to a dairy product whose lactose content has been reduced with respect to its original lactose content. In specific embodiments, the lactose content has been reduced by at least 75%, at least 80%, at least 85%, at least 90%, at least 95%, at least 95.5%, at least 96%, at least 96.5%, at least 97%, at least 97.2%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, or at least 99.9%.

Furthermore, the term "lactose-free dairy product" as used herein relates to a dairy product no longer containing any lactose, or containing lactose to a maximum of 1%, 0.9%, 0.8%, 0.7%, 0.6%, 0.5%, 0.4%, 0.3%, 0.2%, or 0.1% (w/v).

The term "dairy products" as used herein is not particularly limited and includes a type of food produced from or containing the milk of mammals, preferably cattle, water buffaloes, goats, sheep, camels, and humans. In preferred embodiments, the dairy product is milk, preferably bovine milk.

In preferred embodiments, the enzyme having beta-galactosidase activity used in the present invention consists of
(a) the amino acid sequence of SEQ ID NO: 1; or
(b) an amino acid sequence having at least 70% sequence identity to SEQ ID NO: 1 and having beta-galactosidase activity.

The term "enzyme comprising an amino acid sequence having at least 70% sequence identity to SEQ ID NO: 1 and having beta-galactosidase activity" relates to polypeptides that can comprise any number of amino acid substitutions, additions, or deletions with respect to the amino acid sequence of SEQ ID NO: 1, provided that the resulting polypeptides fulfil the requirement of having at least 70% sequence identity to SEQ ID NO: 1 and retain biological activity of a beta-galactosidase. In this context, the term "retains the biological activity of a beta-galactosidase" as used herein relates to polypeptides that have at least 5%, at least 10%, at least 25%, at least 50%, preferably at least 55%, at least 60%, at least 65%, at least 70%, at least 75%, at least 80%, at least 82%, at least 84%, at least 86%, at least 88%, at least 90%, at least 91%, at least 92%, at least 93%, at least 94%, at least 95%, at least 96%, at least 97%, at least 97.5%, at least 98%, at least 98.5%, at least 99%, at least 99.5%, 100%, or more than 100% of the activity of the *P. wynnii* beta-galactosidase having the amino acid sequence of SEQ ID NO: 1, as determined in a standard beta-galactosidase activity assay known in the art.

While the number of amino acid substitutions, additions, or deletions is generally only limited by the above proviso concerning the sequence identity and biological activity of the resulting polypeptide, it is preferable that the resulting polypeptide has at least 75%, at least 80%, at least 85%, at least 90%, at least 95%, at least 97%, at least 98%, at least 98.5%, at least 99%, at least 99.25%, at least 99.5%, at least 99.6%, at least 99.7%, at least 99.75%, at least 99.8%, at least 99.85%, at least 99.9%, or at least 99.95% sequence identity to the amino acid sequence of SEQ ID NO: 1.

Means for determining the sequence identity of an amino acid sequence to a reference sequence are known in the art.

In a second aspect, the present invention relates to a method for the production of a lactose-depleted or lactose-free dairy product, comprising the step of incubating a dairy product with an enzyme having beta-galactosidase activity as defined for the uses of the present invention under conditions and for a duration of time suitable to hydrolyse lactose present in said dairy product.

In this aspect, all relevant definitions and limitations indicated above for the uses of the present invention apply in an analogous manner. In particular, the dairy products and the enzyme having beta-galactosidase activity are as defined above.

Means for reducing the lactose content in dairy products by way of using an enzyme having beta-galactosidase activity, as well as respective reaction conditions and incubation durations are not particularly limited and are known in the art. In specific embodiments, the methods of the present invention are performed at a temperature of 4 to 8 °C and for a duration of 24 to 72 hours.

In preferred embodiments, the lactose content in the lactose-depleted or lactose-free dairy product is at most 1%, 0.9%, 0.8%, 0.7%, 0.6%, 0.5%, 0.4%, 0.3%, 0.2%, or 0.1% (w/v).

In a third aspect, the present invention relates to a dairy product that is produced by a method according to the present invention.

In this aspect, all relevant definitions and limitations indicated above for the uses and methods of the present invention apply in an analogous manner. In particular, the dairy products are as defined above.

In the present invention, a beta-galactosidase derived from *P. wynnii* was produced recombinantly and characterized in detail. Surprisingly and advantageously, this beta-galactosidase is characterized by (i) a high affinity to its substrate lactose, (ii) a lack of product inhibition by galactose, and (iii) a high enzyme activity.

Specifically, by cultivating a recombinant strain, an active beta-galactosidase could be proved. The beta-galactosidase showed a high affinity to the substrate lactose, with a *K*_{M,lactose}-value of 0.63 ± 0.045 mM. The influence of galactose was investigated in Novo buffer (i.e., a buffer which has a similar ion concentration to milk) at 8°C. No inhibition of beta-galactosidase up to a concentration of 200 mM could be detected. During the hydrolysis of lactose in milk with an enzyme activity of 2.7 nkat_{lactose, 8 °C}/mLₘᵢₗₖ, 98.1 % of the lactose had been hydrolysed after 47 h (0.86 g/L). Already after 72 h no more lactose could be detected. During hydrolysis using the industrial enzyme Opti-Lactase (with the same enzyme activity), the lactose content was 10 times higher (9.8 g/L) after 47 h. After 149 h a lactose content of 0.56 g/L could still be detected. During hydrolysis, the synthesis of galactooligosaccharides (GOS) was qualitatively investigated. While Opti-Lactase produced hardly any GOS and only low-molecular saccharides, beta-galactosidase from *P*. *wynnii* synthesized higher-molecular GOS.

For the hydrolysis of lactose in milk to be effective, the affinity of the enzyme to the substrate lactose must be high, *i.e.,* the *K*_{M} value must be low. The beta-galactosidase from *P. wynnii* used in the present invention possesses a lower *K*_{M} value than the beta-galactosidases known in the art and used as technical enzyme preparations (cf. Table 1).

**Table 1: Overview of K_{M,lactose}-values from different β-galactosidases**

| **Source of beta-galactosidase** | ***K*_{M,lactose}** | **Buffer system** | **Temperature [°C]** |
|---|---|---|---|
| *P. wynnii* | 0.63 | milk + Novo buffer | 8 |
| | 0.34-0.52 | Novo-buffer | |
| *Aspergillus oryzae* | 51.5-52.1 | Acetate buffer | 35 |
| *Bacillus circulans* | 16 | phosphate buffer | 40 |
| *K. lactis* | 30.8 | milk + water | 8 |
| | 19.8 | phosphate buffer | 37 |
| | 33.5 | milk + Novo buffer | 8 |
| Metagenome M1 | 2.10 | milk + Novo buffer | 8 |
| | 1.44 | Novo buffer | |
| *P. barengoltzii* | 43.27 | MOPS buffer | 45 |
| *P. thiaminolyticus* | 206 | phosphate buffer | 37 |

In addition the beta-galactosidase from *P. wynnii* used in the present invention is not inhibited by the hydrolysis-generated product galactose which leads to a more effective hydrolysis of lactose (cf. Table 2)

**Table 2: Influence of galactose on the enzyme activity of different beta-galactosidases**

| **Source of beta-galactosidase** | **Influence of galactose (200 m*M*)** | **Temperature [°C]** | **Substrate** |
|---|---|---|---|
| *P. wynnii* | 100 % EA | 8 | Lactose |
| *K. lactis* | 32.5 % EA | 8 | Lactose |
| | 75 % EA | 37 | *oNPGal* |
| Metagenome M1 | 100 % EA | 37 | *oNPGal* |
| *P. barengoltzii* | 43.27 % EA | 45 | *oNPGal* |

During lactose hydrolysis in milk (scale of -30 mL), which was carried out at 5 to 8 °C using beta-galactosidase from *P*. *wynnii* according to the present invention and Opti-Lactase (*K. lactis*) over a period of 149 h, lactose was degraded significantly faster using beta-galactosidase from *P. wynnii.* After 47 h, 98.1 % had been hydrolysed. This corresponded to a concentration of 0.86 g/L. After 72 h no more lactose could be detected by HPLC (detection minimum 0.45 g/L). In comparison to this, using Opti-Lactase, after 47 h a ten times higher lactose concentration was present (9.8 g/L). After 149 h a lactose concentration of 0.56 g/L could be determined (cf. the Examples, below).

The present invention discloses the following amino acid sequences:
*P. wynnii* beta-galactosidase (SEQ ID NO: 1) 
MRKKLVYSPP TNGYPEWNNN PECFQINRMD AHATWIPFNT TEDALLGDPQ SSPNYLSLNG
MWKFAYAETP DQRIRNFFEK NYDCSSWAEL NVPSHWQMHG YDYPQYTNVR YPWSEREPEL
KPPFAPTQYN PVGSYVRTFS VPEDWSGKPV FISFQGVESA FYVWLNGELV GYSEDTFTPA
EFDLTPYLIP GDNKLAVEVY RWCDASWLED QDFWRLSGIF RDVYLYTTPE AHIYDFFVRT
ELDEQYRHAE LQLDVKLMDY FERTAEAVIV HAQLYDHDQN AIFDQPLSQT VYFNSASTQT
LQFSSSIIDP LKWSAEHPNL YTLVLSLHHV DGELMEAVSC RVGFRTFELK DGLMKINGKR
IVFKGVNRHE FSCDTGRSID VDDMVRDILL MKAHNINAVR TSHYPNQTIW YDLCDQYGLY
VIDETNLETH GSWSYGQTDL GGNTVPGSRP EWRANVLDRC NSMLQRDKNH PSIVIWSLGN
ESFGGDNFVA MHDFLKKEDP SRLVHYEGLF HYRESDVASD IESTMYISPA DVEQYALNDP
KKPYILCEYS HAMGNSCGGL HLYWEVFEKY DILQGAFIWD WIDQAIRLKQ ADGSMHMAYG
GDFGESPHDG NFCGNGLIFA DRSVSPKLYE VKKCYQNVKF EAVDLERGIY RVTNQNLFTD
LAEYALAWEV SCNGNPVLKG TVDLAVPAGE SAEISVPVVD EPNLQSEGEH VLTFSLQLKK
STLWADAGHE VAWEQFLLPT PQFMAGQDQD SVLTSDRGVI VEEQAGRLTV QAADVSLQFS
TSSGYLISMQ NKGKELLLEP VRPNFWRAVT DNDLGNKHHE RCAVWNTAGA GCTLASFESH
KNVDGVTVRA KYTVPTVPIS SLILEYRIQE NGSIEVFEEL SPGMGLPEIP EIGLMFIVED
RLDTVSWYGR GPHENYWDRK TGARLGYFSG SVQDQFVPYI RPQECGNKTD VRFASITGGI
NGSGFRVDGD PVLELNALPW TPAELEANDH IYKLPASNKT VVRVNYKQMG VGGDNSWGAT
*P. wynnii* beta-galactosidase coding sequence (SEQ ID NO: 2)

atgcgtaaaa aacttgtcta ttcaccccct accaacgggt acccggaatg gaataataat
cccgaatgtt ttcagatcaa ccgaatggat gcccatgcta catggatacc ttttaataca
acagaggatg ccttgcttgg agatccacaa tcctcgccca actatttgtc tttgaatggg
atgtggaaat tcgcctatgc agagactccg gatcaacgca tacgtaactt ttttgaaaaa
aactatgatt gcagttcttg ggctgaactg aacgttccct ctcattggca gatgcatgga
tatgactacc ctcagtatac gaatgtacgg tatccctgga gtgaacgtga gccggagctc
aaaccgcctt ttgctcccac ccaatataat ccggttgggt cttatgtacg aaccttctct
gtaccggagg actggagcgg gaagcctgtt ttcatcagct ttcaaggtgt ggagtctgcc
ttctatgttt ggctcaatgg agagttggtc ggatacagcg aggatacctt cacccctgcg
gaatttgact taactcctta tctaatcccg ggagacaata agcttgccgt tgaagtgtat
cgttggtgtg acgccagttg gctggaggat caggatttct ggagacttag cggcatattt
agagatgtct atctatatac aacaccggaa gcccatattt atgatttctt tgttcgaaca
gaactggatg agcagtaccg gcatgctgaa ttgcagctgg atgtgaagct tatggattat
tttgaaagaa ccgctgaggc agttattgtg catgcgcagc tttatgatca cgatcagaac
gctatctttg atcagccgct ttcacagaca gtctacttca atagcgcttc tacccagacg
ctccaattct cttcttctat tattgacccg ttaaaatgga gtgcggaaca tccgaatcta
tatacgttag tgttgtcttt acatcatgtg gatggagagc tcatggaagc cgttagctgc

cgtgttggct ttcgtacatt cgagctaaag gacggtttaa tgaagattaa tggaaaacgg
atcgtcttta agggcgtgaa ccgccatgaa ttctcatgcg ataccggcag atcaattgat
gtcgatgata tggtacggga cattctactg atgaaagcac acaacatcaa tgccgttcgg
acttcgcatt atccgaatca gaccatatgg tatgaccttt gtgaccaata tgggctgtat
gtaattgatg agaccaatct ggagacacat ggttcgtgga gctatggaca gacagactta
ggcggaaata cggtacccgg cagtagacct gaatggcgcg cgaatgtact ggatcggtgt
aattcaatgc tccaaaggga caaaaaccac ccttccatcg tcatctggtc actcggtaac
gagtcctttg gcggtgataa ctttgtggct atgcatgatt ttcttaagaa agaagacccc
tcccggcttg tccattatga aggcctcttt cactatcgcg agagcgatgt tgcctcggac
attgagagca ccatgtacat tagtccggcc gatgtggaac agtatgcgct taatgatcca
aagaagcctt atattctatg cgagtatagc catgcaatgg gcaactcctg cgggggattg
catctatact gggaagtttt tgagaaatat gatattttac agggtgcctt tatctgggac
tggattgatc aagcgattcg acttaagcaa gccgacggat ccatgcatat ggcctacggc
ggagacttcg gggaatcccc tcatgacggc aacttctgcg gaaatggctt gattttcgct
gaccgttcag tatctcctaa gctgtatgag gtcaagaaat gctaccagaa tgttaaattt
gaggctgtcg atttagagcg aggcatttat cgggtaacca atcagaatct atttacggat
ttggcggagt acgcactggc ttgggaagta agctgtaacg gcaatcctgt gcttaagggt
actgttgatc tggctgtccc tgcaggagaa tcggcagaaa tttcggttcc cgtggtcgat
gaacctaacc tgcaatccga gggcgagcat gtcctcacgt ttagcctgca attgaagaaa
tccacccttt gggcagacgc aggccatgaa gtggcatggg aacaattcct gctgcctaca
cctcagttta tggcgggtca ggatcaagac tctgttttga catcagatcg tggtgtgatt
gtggaggaac aagcagggcg tttaactgtt caggcggcgg atgtctccct tcaattcagt
acttcaagcg gttatctgat ctctatgcaa aataaaggca aggaactgct gctcgaacct
gttcgtccga atttctggcg tgcagttacg gataacgatc tagggaacaa acaccatgag
cgttgcgcgg tttggaatac agcgggagct ggctgtacac tagcttcctt tgaatcgcat
aagaatgtag atggcgtaac tgttcgtgct aagtacaccg tacccaccgt acccatttct
tcactgatat tggaatatag aatccaagaa aatggctcca ttgaagtctt tgaagagcta
tcaccgggca tgggcttgcc ggagattcct gaaattggcc tcatgtttat cgttgaagat
cgactcgaca ccgtttcatg gtatggccgc ggaccgcatg agaattactg ggaccggaag
acaggcgcca gacttggata tttttcggga agtgtccagg atcagttcgt tccgtatata
aggcctcaag aatgcggaaa caaaacggat gtgcgttttg ccagtatcac tgggggtata
aacggctcag gattccgtgt ggatggcgat cctgtactgg aactaaatgc cttgccttgg
acacccgctg agttggaagc gaatgatcac atatataagc tgccagccag taataaaacc
gttgtgcgtg tgaactacaa gcaaatgggt gtaggtggag acaacagctg gggcgcgacg
acacatcctg aattcacttt gccggcggat caaacttatg ggtttcggtt tacgattcgg
atggtgtaa

The figures show:
Figure 1:
   Determination of the effect on the enzyme activity of the beta-galactosidase from *P. wynnii* and Opti-Lactase using different galactose concentrations (125 m*M* and 200 m*M*) as well as different lactose concentrations (125 mM and 50 m*M*). The enzyme activity was measured with Novo-buffer (pH 6.7) and lactose as substrate at 8 °C.
Figure 2:
   Hydrolysis of lactose in milk (31.5 mL scale) with the *P. wynnii* beta-galactosidase and the commercial beta-galactosidase Opti-Lactase. Δ - no lactose content could be determined.
Figure 3:
   TLC analysis of the hydrolysis products of milk by the beta-galactosidase from *P*. *wynnii* (A) and by the commercial Opti-Lactase (B).

The present invention will be further illustrated by the following example without being limited thereto.

### Examples

### Example 1:

### Cloning of Paenibacillus wynnii beta-galactosidase

The construction of the expression vector for the beta-galactosidase from *Paenibacillus wynnii* was based on the genomic DNA sequence of the beta-galactosidase from *Paenibacillus wynnii* DSM18334 (European Molecular Biology Laboratory: EMBL coding: KGE19535.1; SEQ ID NO: 2) available at the EMBL, European Bioinformatics Institute. One nucleotide was exchanged for cloning, but this did not result in an amino acid exchange. In general, standard molecular biology methods according to Sambrook and Russell (Sambrook, J.; Russell, D. W. Molecular Cloning: A Laboratory Manual; Cold Spring Harbour Laboratory Press: New York, NY, 2001; Vol. 1) were used. Cloning in pET-20b (+) was followed by transformation in *E. coli* XL-1 blue cells to introduce a C-terminal His6-tag in frame, resulting in the construct pET-20b-P.w.-beta-gal. For the expression of the *P. wynnii* beta-galactosidase, the construct was transformed in *E. coli* BL21(DE3) cells.

### Example 2:

### Cultivation of E. coli BL21(DE3) pET20b-Paenibacillus wynnii-beta-galactosidase

*E. coli* BL21(DE3)-pET20b-P.w.-beta-gal was cultivated in 2YT_{Amp} medium (16 g L⁻¹ tryptone, 10 g L⁻¹ yeast extract, 5 g L⁻¹ NaCl containing 100 *µ*g mL⁻¹ ampicillin concentration) with 2 % glucose (w/v) in a bioreactor with 3.5 L operating volume. Before inoculating the bioreactor different precultures were prepared. A single colony of *E. coli* BL21(DE3)-pET20b-*P.w*.-beta-gal was picked from a LB agar plate and transferred into a prepared test tube containing 5 mL 2YT_{Amp} medium with 2 % (w/v) glucose and incubated on an orbital shaker at 120 rpm for 15 h (preculture I). Preculture I was transferred after 15 h into a baffled flask containing 30 mL 2YT_{Amp} medium and 2 % (w/v) glucose (preculture II). Preculture II was incubated on an orbital shaker at 90 rpm for 15 h before it was added to a baffled flask containing 2YT_{Amp} medium with 2 % (w/v) glucose (preculture III). After incubation for 15 h on an orbital shaker (80 rpm) preculture III was added sterilely to 3.5 L 2YT_{Amp} medium with 2 % (w/v) glucose that was prepared in a bioreactor. The following parameters were chosen for the cultivation: 300 rpm, air gassing *vvm* = 2, and pH 7. The pH was adjusted using 2 M NaOH and 2 *M* H₃PO₄. The cells were cultivated at 37 °C up to an OD₆₀₀ of 5. For the expression of the recombinant protein the cells were cooled down to 30 °C and induced with 0.5 mM isopropyl-beta-D-1-thiogalactopyranoside (IPTG). After further 9 h cultivation at 30 °C the cells were harvested using a continuous working centrifuge (CEPA Rapid centrifuge Type GLE, flow rate 350 mL min⁻¹, 36,000 g, 4 °C). The harvested cells were washed twice with saline and centrifuged for 10 min at 6,000 g and 4 °C.

100 m*M* potassium phosphate buffer with 5 mM MgCl₂ (pH 6.75) was added to the cells to prepare a 25 % (w/v) cell suspension. The cells were disrupted on ice using a sonificator (cycle 0.5, amplitude 95 %). Cell disruption was carried out in ten cycles of one-minute pulsed intervals followed by one-minute breaks. After disruption the cell suspension was centrifuged for 20 min at 8000 g and 4 °C. The cell pellet was discarded and the enzyme in the supernatant was used for further experiments.

### Example 3:

### Purification of the recombinant P. wynnii beta-qalactosidase

The recombinant *P*. *wynnii* beta-galactosidase with a hexa histidine-tag was purified by nickel affinity chromatography (Biofox 40 IDALow XK16, 5 mL, Knauer, Berlin). The disrupted cells were centrifuged for 20 min at 4 °C and 8,000 *g.* The supernatant was centrifuged again for 20 min at 4 °C and 20000 g. Afterwards, the supernatant was filtrated (0.45 *µ*m) and 5 mL of the crude extract were injected into an ÄKTA FPLC system (GE Healthcare, Freiburg). The column was previously equilibrated with 2 CV binding buffer. The conditions for the purification of *P*. *wynnii* beta-galactosidase are listed in Table 3. The eluted proteins were detected by an UV detector at *λ* = 280 nm and fractionated in 2.5 mL fractions. The fractions in which an UV signal was detected were pooled. Size exclusion chromatography columns (PD10 columns, GE Healthcare, Freiburg) were used to change the buffer to 100 mM potassium phosphate buffer (pH 6.75 containing 5 m*M* magnesium chloride).

**Table 3: Conditions for the purification of P. wynnii beta-galactosidase by nickel affinity chromatography**

| | |
|---|---|
| Flow rate - binding | 1 mL/min |
| Flow rate - elution | 2 mL/min |
| Elution gradient | 2 CV 10 m*M* imidazole |
| | 4 CV linear 10 - 250 m*M* imidazole |
| Washing | 2 CV elution buffer |

| | |
|---|---|
| Binding buffer: 100 m*M* potassium phosphate buffer containing 150 m*M* NaCl Elution buffer: 100 m*M* potassium phosphate buffer containing 150 m*M* NaCl and 250 mM imidazole | |

### Example 4:

### Determination of enzyme activity

The beta-galactosidase activity was determined using either the synthetic substrate o-nitrophenyl-beta-D-galactopyranoside (*o*NPGal) or the natural substrate of the enzyme lactose.

### Beta-galactosidase activity with o-nitrophenyl-β-D-galactopyranoside (oNPGal) as substrate.

The enzyme activity of beta-galactosidase was determined in 1.2 mL scale using *o*NPGal as substrate at 37 °C. For the assays a final concentration of 25 m*M o*NPGal dissolved in 100 m*M* potassium phosphate buffer (pH 6.75 containing 5 mM magnesium chloride) was used. Substrate and enzyme solution were preheated separately. The reaction was performed in a temperature-controlled cuvette using a spectrophotometer by adding enzyme solution to *o*NPGal solution. The increase of absorbance at 405 nm as result of o-nitrophenol release was measured for 2 min. The activity was calculated from the slope of the straight line.

One nanokatal is defined as the amount of enzyme that catalyses the release of 1 nmol of o-nitrophenol from oNPGal per second. The amount of the released *o-*nitrophenol was determined using a calibration curve (range: 0.0167-1.67 m*M o-*nitrophenol).

### Beta-galactosidase activity with lactose as substrate.

The beta-galactosidase activity was determined using lactose as a substrate. Beta-Galactosidase hydrolyses the beta-1,4-glycosidic bond in lactose and release D-glucose and D-galactose.

For the substrate solution 400 m*M* lactose was dissolved in Novo buffer (the composition of which is depicted in Table 4). The beta-galactosidase activity was determined in a thermo shaker in 1.5 mL reaction tubes at 8°C. The enzyme mix and the lactose solution were preincubated at 8 °C for 12 min. To start the enzymatic reaction 100 *µ*L of the enzyme mix were added to 100 *µ*L lactose solution. The reaction was carried out for 5 min at 8 °C. The reaction was stopped by adding 300 *µ*L 1 M HClO4 to the enzyme-substrate-mix. The mix was kept on ice for 5 min before it was centrifuged at 13 000 rcf and 4 °C for 5 min. 160 *µ*L of the supernatant were transferred to 75 *µ*L 2 *M* KOH to neutralise the solution. The neutralisation step was checked using pH paper and the pH was adjusted if necessary. The solution was kept on ice for another 5 min before it was centrifuged at 13 000 rcf at 4 °C for 5 min. All assays were performed in triplicate.

**Table 4: Composition of Novo buffer**

| **Component** | **Concentration [mM]** |
|---|---|
| Trisodium citrate | 2.7 |
| Citric acid | 7.91 |
| K₂HPO₄ | 2.99 |
| KH₂PO₄ | 10.84 |
| KOH | 19.43 |
| MgCl₂ | 4.08 |
| CaCl₂ | 5.1 |
| Na₂CO₃ | 3.33 |

### Enzymatic D-glucose determination.

The glucose concentration was determined for the calculation of the beta-galactosidase activity with lactose as a substrate. The glucose concentration was determined using an enzymatic assay. The commercial kit hexokinase/glucose-6-phosphate dehydrogenase (Megazyme, Wicklow, Ireland) was used. The D-glucose is phosphorylated to glucose-6-phosophate by the enzyme hexokinase using ATP as cosubstrate. In a coupled reaction glucose-6-phosphate is oxidized to gluconate-6-phosphate by the enzyme glucose-6-phosphate dehydrogenase while NADP⁺ is reduced to NADPH which was quantified photometrically at 340 nm. The amount of the formed NADPH equals the amount of glucose in the solution. The determination of the glucose concentration was performed using a robotic liquid handling system (MultiPROBE II EX, Packard Bioscience, Meriden, CT, USA). The hexokinase/glucose-6-phosphate dehydrogenase suspension was diluted 1:4 with H₂O_{dd} before use. TRA buffer which contained NADP⁺ and ATP was prepared according to Table 5.

**Table 5: Composition of TRA buffer for the enzymatic determination of D-glucose**

| **Component** | **Concentration [g/L]** |
|---|---|
| Triethanolamine hydrochloride | 33.6 |
| ATP | 2.15 |
| NADP⁺ | 1.3 |
| MgSO₄ · 7 H₂O | 0.9 |

For the glucose determination 235 *µ*L TRA buffer were prepared in a 96 well plate and 15 *µ*L of the sample solution was added. After 3 min the absorption at *λ* = 340 nm was measured in a plate reader (A1). Subsequently, 5 *µ*L of the diluted hexokinase/glucose-6-phosphate dehydrogenase were added to each well. The plate was shaken in the plate reader and incubated at room temperature. After 30 min the absorption at *λ* = 340 nm was measured again (A2). With the absorption difference ΔA = A2 - A1 the glucose concentration was calculated using a calibration curve (range; 0.0075-1 g/L glucose).

### Example 5:

### Determination of kinetic parameters

The kinetic parameters were determined using the natural substrate lactose. The reaction matrices and conditions were chosen in order to simulate conditions in milk systems. The chosen reaction matrices were Novo buffer (cf. Table 4, above) and milk diluted with Novo buffer. The reaction temperature was 8 °C and the pH value was 6.7.

### Determination of K_{M}.

For the determination of the *K*_{M} values of *P*. *wynnii* beta-galactosidase lactose concentrations ranged from 0.76 mM to 97.5 m*M*. In order to obtain different lactose concentrations in milk, milk was diluted with Novo buffer. It was used a beta-galactosidase activity of 6.11 nkat_{Lactose,8 °C}/mL for the determination in milk and 11.34 nkat_{Lactose,8 °C}/mL in buffer. The enzyme activities were measured under initial reaction velocity conditions of the enzyme. The reaction times for the initial velocity of the enzymes were determined in previous experiments. The kinetic parameter *K*_{M} was analysed using the Enzyme Kinetics Module in SigmaPlot 12.5 (Systat Software, Inc., San Jose, USA).

The *K*_{M} values determined are summarized in Table 6. In the case of the studies in Novo buffer the enzyme activity decreased again with increasing lactose concentration. For this reason, in addition to the Michaelis Menten evaluation, the evaluation also included an evaluation taking into account a substrate inhibition.

**Table 6: Summary of the determined K_{M} values using the Enzyme Kinetics Module in SigmaPlot 12.5. A: Evaluation according to Michaelis-Menten, B: Evaluation under consideration of substrate inhibition.**

| | ***K*_{M,Lactose} [m*M*]** | **R² [-]** |
|---|---|---|
| Milk (A) | 0.63 ± 0.045 | 0.95 |
| Novo buffer (A) | 0.34 ± 0.084 | 0.61 |
| Novo buffer (B) | 0.52 ± 0.089 | 0.80 |

### Influence of galactose on the enzyme activity.

The influence of galactose was investigated with *P. wynnii* beta-galactosidase and the commercial beta-galactosidase Opti-Lactase (Optiferm GmbH, Oy-Mittelberg (D)). The determination of the enzyme activity in Novo buffer was conducted with a lactose concentration of 125 m*M* and 50 m*M* respectively. To this solutions 125 or 200 m*M* galactose were added. The determination of the enzyme activity was performed as described above.

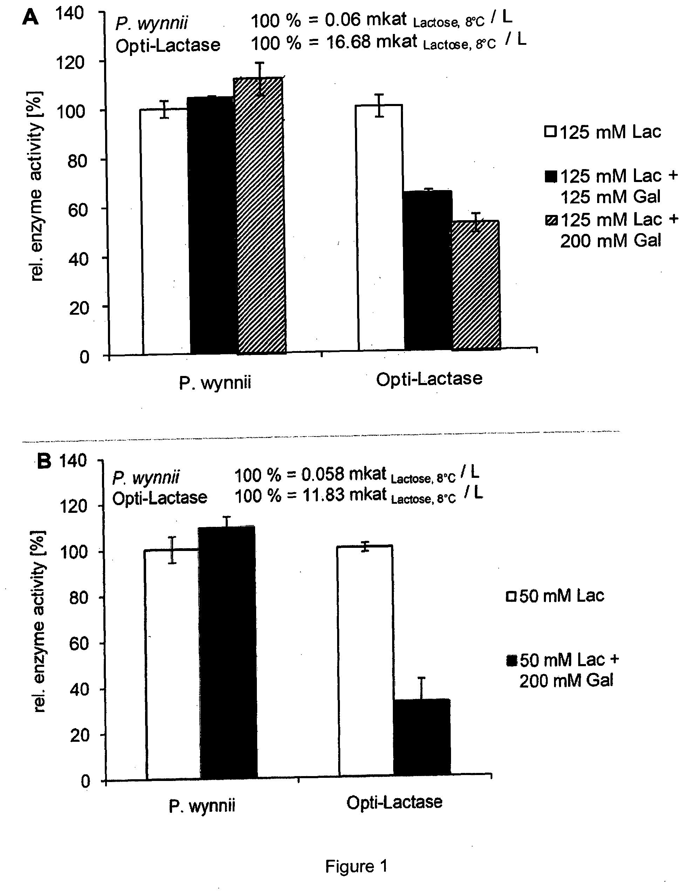

Figure 1 shows the relative enzyme activity of the two beta-galactosidases. First, 125 and 200 mM galactose respectively were added to 125 m*M* lactose (A). The enzyme activity of the technical enzyme preparation decreased with increasing galactose concentration. An activity of 51.8 % was determined. In contrast, galactose did not have an inhibitory effect on the activity of the beta-galactosidase from *P*. *wynnii.* In another experiment 200 m*M* galactose was added to 50 m*M* lactose (B). Here, the same effect as in (A) could be observed. Furthermore, the comparison of enzyme activity at 125 and 50 mM showed that with lower lactose concentrations the activity of Opti-Lactase decreased significantly. The determined enzyme activity decreased from 16.68 ± 0.76 mkat_{Lactose,8 °C}/L to 11.84 ± 0.23 mkat_{Lactose,8 °C}/L. The enzyme activity of the beta-galactosidase from *P. wynnii* did not change significantly (59.9 ± 2.0 µkat_{Lactose,8 °C}/L).

### Example 6:

### Comparison of lactose hydrolysis of P. wynnii beta-galactosidase and commercial preparations of Opti-Lactase

The hydrolysis of lactose in milk was performed both with the beta-galactosidase from *P*. *wynnii* and with the technical enzyme preparation Opti-Lactase (*K. lactis*). The hydrolysis was conducted in a 31.5 mL scale at approx. 5 to 8 °C. For the conversion an enzyme activity of 2.7 nkat_{Lactose, 8 °C}/mL milk was used. Samples were taken over a period of 149 h. Therefore 200 µL samples were taken and stopped with perchloric acid as described above and the lactose content was determined by HPLC (cf. Example 7, below). GOS synthesis was investigated by HPLC and thin-layer chromatography (cf. Example 8, below).

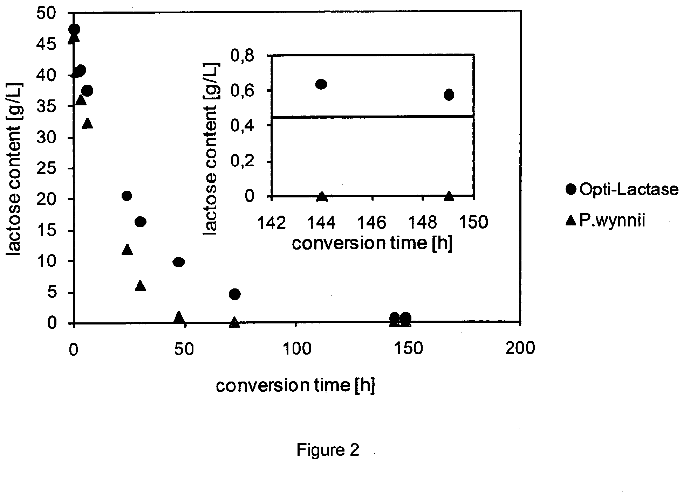

Figure 2 shows the course of the lactose hydrolysis in milk over time. The beta-galactosidase from *P. wynnii* exhibited a better performance in lactose hydrolysis from the beginning. After 24 hours, 75% of the lactose had already been hydrolysed with *P. wynnii* beta-galactosidase compared to 56% with Opti-Lactase. After 47 h 98.1 % lactose was hydrolysed which corresponds to a residual milk lactose content of 0.86 g/L with P. wynnii beta-galactosidase and after 72 h no more lactose could be detected by HPLC (detection limit 0.45 g/L). In comparison to *P*. *wynnii* beta-galactosidase, with Opti-Lactase lactose amounts of 9.8 g/L after 47 h, 4.6 g/L after 72 h, and 0.56 g/L even after 149 h could be determined.

### Example 7:

### HPLC analysis

An Agilent Series 1100 HPLC system, equipped with a Shodex HILICpak VG-50 4E column (4.6 × 250 mm, 5 *µ*m, Shodex) and a low-temperature evaporative light scattering detector (ELSD Sedex 85LT, Sedere, Alfort ville Cedex, France) at 50 °C and 3 bar was used to determine the concentration of lactose. The column was eluted with 75 % (v/v) acetonitrile, 15% (v/v) methanol, and 10% (v/v) double-distilled water at a flow rate of 0.75 mL/min at 40 °C.

### Example 8:

### Thin layer chromatography

Thin layer chromatography was used in order to visualize the products (lactose, glucose, galactose and galactooligosaccharides (GOS)) generated from milk lactose by enzymatic conversion with the two beta-galactosidases (cf. Example 6, above). Aliquots of 1 *µ*L of the sample prepared were spotted on TLC plates (Silica gel 60F254, Merck, Whitehouse Station, NJ), and developed in a solvent system of 1-butanol/2-propanol/water (3:12:4). The plates were developed by a colouring agent (6.5 m*M* N-1-naphthyl ethylenediamine dihydrochloride and 3 % sulfuric acid in methanol) at 100 °C until the spots became visible.

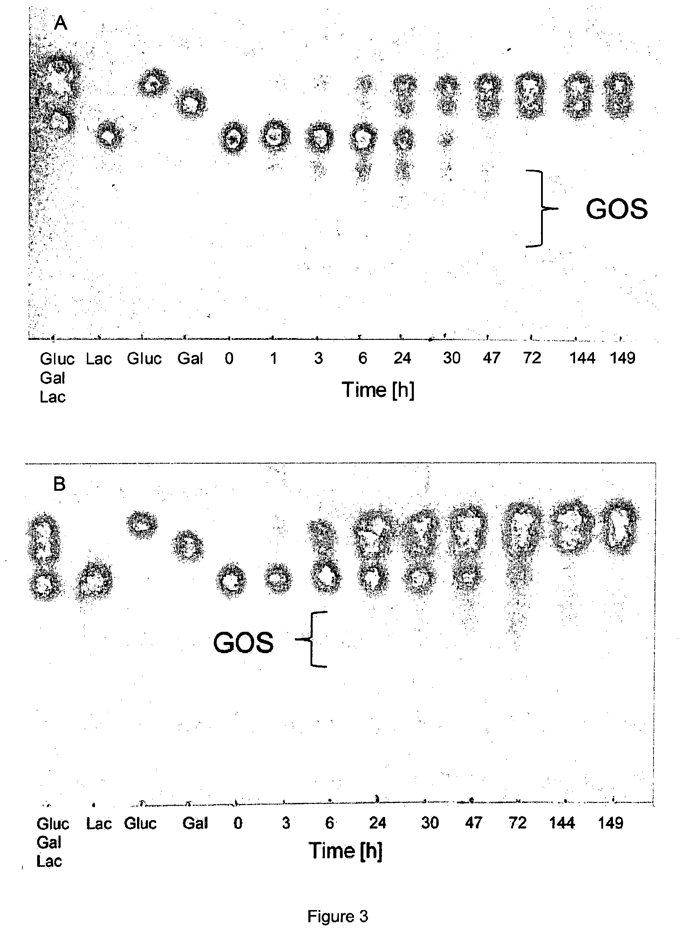

Figure 3A shows the HPLC analysis of the samples from lactose hydrolysis by means of Opti-Lactase and Figure 3B the analysis using beta-galactosidase from *P. wynnii.* In addition to the decrease in lactose and the increase in galactose or glucose, the formation of GOS over time can be observed. It was obvious that the GOS synthesis during the hydrolysis of lactose with Opti-Lactase occurred much later than during the hydrolysis with *P. wynnii* beta-galactosidase. While during the conversion of lactose by Opti-Lactase only a minimal GOS formation could be detected after 24 h, the conversion by *P. wynnii* beta-galactosidase showed the formation of GOS after 1 h already. The highest yield of GOS was approximately 72 h for the conversion with Opti-Lactase and 24 h with *P. wynnii* beta-galactosidase. However, the yield of Opti-Lactase was qualitatively lower than that of *P. wynnii* beta-galactosidase. In addition, different GOS were formed. While Opti-Lactase synthesized rather low molecular weight GOS, *P. wynnii* beta-galactosidase generated also higher molecular weight GOS.

## Claims

1. Use of an enzyme having beta-galactosidase activity in the production of lactose-depleted or lactose-free dairy products, wherein said enzyme comprises
(a) the amino acid sequence of SEQ ID NO: 1; or
(b) an amino acid sequence having at least 70% sequence identity to SEQ ID NO: 1 and having beta-galactosidase activity.

2. The use according to claim 1, wherein the enzyme consists of
(a) the amino acid sequence of SEQ ID NO: 1; or
(b) the amino acid sequence having at least 70% sequence identity to SEQ ID NO: 1 and having beta-galactosidase activity.

3. The use according to claim 1 or claim 2, wherein the amino acid sequence in (b) has at least 75%, at least 80%, at least 85%, at least 90%, at least 95%, at least 97%, at least 98%, at least 98.5%, at least 99%, at least 99.25%, at least 99.5%, at least 99.6%, at least 99.7%, at least 99.75%, at least 99.8%, at least 99.85%, at least 99.9%, or at least 99.95% sequence identity to SEQ ID NO: 1.

4. The use according to claim 1 or claim 2, wherein the enzyme consists of the amino acid sequence of SEQ ID NO: 1.

5. The use according to any one of claims 1 to 4, wherein the dairy product is milk.

6. A method for the production of a lactose-depleted or lactose-free dairy product, comprising the step of incubating a dairy product with an enzyme having beta-galactosidase activity as defined in any one of present claims 1 to 4 under conditions and for a duration of time suitable to hydrolyse lactose present in said dairy product.

7. The method according to claim 6, wherein the dairy product is milk.

8. The method according to claim 6 or claim 7, wherein the lactose content in the lactose-depleted or lactose-free dairy product is at most 0.1% (w/v).


SEQUENCE LISTING

<110>  Universität Hohenheim

<120>  Paenibacillus wynnii beta-galactosidase for the production of
       lactose-depleted dairy products

<130>  H 3780EU

<160>  2

<170>  PatentIn version 3.5

<210>  1
<211>  1042
<212>  PRT
<213>  Paenibacillus wynnii

<400>  1

Met Arg Lys Lys Leu Val Tyr Ser Pro Pro Thr Asn Gly Tyr Pro Glu
1               5                   10                  15

Trp Asn Asn Asn Pro Glu Cys Phe Gln Ile Asn Arg Met Asp Ala His
            20                  25                  30

Ala Thr Trp Ile Pro Phe Asn Thr Thr Glu Asp Ala Leu Leu Gly Asp
        35                  40                  45

Pro Gln Ser Ser Pro Asn Tyr Leu Ser Leu Asn Gly Met Trp Lys Phe
    50                  55                  60

Ala Tyr Ala Glu Thr Pro Asp Gln Arg Ile Arg Asn Phe Phe Glu Lys
65                  70                  75                  80

Asn Tyr Asp Cys Ser Ser Trp Ala Glu Leu Asn Val Pro Ser His Trp
                85                  90                  95

Gln Met His Gly Tyr Asp Tyr Pro Gln Tyr Thr Asn Val Arg Tyr Pro
            100                 105                 110

Trp Ser Glu Arg Glu Pro Glu Leu Lys Pro Pro Phe Ala Pro Thr Gln
        115                 120                 125

Tyr Asn Pro Val Gly Ser Tyr Val Arg Thr Phe Ser Val Pro Glu Asp
    130                 135                 140

Trp Ser Gly Lys Pro Val Phe Ile Ser Phe Gln Gly Val Glu Ser Ala
145                 150                 155                 160

Phe Tyr Val Trp Leu Asn Gly Glu Leu Val Gly Tyr Ser Glu Asp Thr
                165                 170                 175

Phe Thr Pro Ala Glu Phe Asp Leu Thr Pro Tyr Leu Ile Pro Gly Asp
        180                 185                 190

Asn Lys Leu Ala Val Glu Val Tyr Arg Trp Cys Asp Ala Ser Trp Leu
    195                 200                 205

Glu Asp Gln Asp Phe Trp Arg Leu Ser Gly Ile Phe Arg Asp Val Tyr
    210                 215                 220

Leu Tyr Thr Thr Pro Glu Ala His Ile Tyr Asp Phe Phe Val Arg Thr
225                 230                 235                 240

Glu Leu Asp Glu Gln Tyr Arg His Ala Glu Leu Gln Leu Asp Val Lys
                245                 250                 255

Leu Met Asp Tyr Phe Glu Arg Thr Ala Glu Ala Val Ile Val His Ala
            260                 265                 270

Gln Leu Tyr Asp His Asp Gln Asn Ala Ile Phe Asp Gln Pro Leu Ser
        275                 280                 285

Gln Thr Val Tyr Phe Asn Ser Ala Ser Thr Gln Thr Leu Gln Phe Ser
    290                 295                 300

Ser Ser Ile Ile Asp Pro Leu Lys Trp Ser Ala Glu His Pro Asn Leu
305                 310                 315                 320

Tyr Thr Leu Val Leu Ser Leu His His Val Asp Gly Glu Leu Met Glu
                325                 330                 335

Ala Val Ser Cys Arg Val Gly Phe Arg Thr Phe Glu Leu Lys Asp Gly
            340                 345                 350

Leu Met Lys Ile Asn Gly Lys Arg Ile Val Phe Lys Gly Val Asn Arg
        355                 360                 365

His Glu Phe Ser Cys Asp Thr Gly Arg Ser Ile Asp Val Asp Asp Met
    370                 375                 380

Val Arg Asp Ile Leu Leu Met Lys Ala His Asn Ile Asn Ala Val Arg
385                 390                 395                 400

Thr Ser His Tyr Pro Asn Gln Thr Ile Trp Tyr Asp Leu Cys Asp Gln
                405                 410                 415

Tyr Gly Leu Tyr Val Ile Asp Glu Thr Asn Leu Glu Thr His Gly Ser
            420                 425                 430

Trp Ser Tyr Gly Gln Thr Asp Leu Gly Gly Asn Thr Val Pro Gly Ser
        435                 440                 445

Arg Pro Glu Trp Arg Ala Asn Val Leu Asp Arg Cys Asn Ser Met Leu
    450                 455                 460

Gln Arg Asp Lys Asn His Pro Ser Ile Val Ile Trp Ser Leu Gly Asn
465                 470                 475                 480

Glu Ser Phe Gly Gly Asp Asn Phe Val Ala Met His Asp Phe Leu Lys
                485                 490                 495

Lys Glu Asp Pro Ser Arg Leu Val His Tyr Glu Gly Leu Phe His Tyr
            500                 505                 510

Arg Glu Ser Asp Val Ala Ser Asp Ile Glu Ser Thr Met Tyr Ile Ser
        515                 520                 525

Pro Ala Asp Val Glu Gln Tyr Ala Leu Asn Asp Pro Lys Lys Pro Tyr
    530                 535                 540

Ile Leu Cys Glu Tyr Ser His Ala Met Gly Asn Ser Cys Gly Gly Leu
545                 550                 555                 560

His Leu Tyr Trp Glu Val Phe Glu Lys Tyr Asp Ile Leu Gln Gly Ala
                565                 570                 575

Phe Ile Trp Asp Trp Ile Asp Gln Ala Ile Arg Leu Lys Gln Ala Asp
            580                 585                 590

Gly Ser Met His Met Ala Tyr Gly Gly Asp Phe Gly Glu Ser Pro His
        595                 600                 605

Asp Gly Asn Phe Cys Gly Asn Gly Leu Ile Phe Ala Asp Arg Ser Val
    610                 615                 620

Ser Pro Lys Leu Tyr Glu Val Lys Lys Cys Tyr Gln Asn Val Lys Phe
625                 630                 635                 640

Glu Ala Val Asp Leu Glu Arg Gly Ile Tyr Arg Val Thr Asn Gln Asn
                645                 650                 655

Leu Phe Thr Asp Leu Ala Glu Tyr Ala Leu Ala Trp Glu Val Ser Cys
            660                 665                 670

Asn Gly Asn Pro Val Leu Lys Gly Thr Val Asp Leu Ala Val Pro Ala

675                 680                 685

Gly Glu Ser Ala Glu Ile Ser Val Pro Val Val Asp Glu Pro Asn Leu
    690                 695                 700

Gln Ser Glu Gly Glu His Val Leu Thr Phe Ser Leu Gln Leu Lys Lys
705                 710                 715                 720

Ser Thr Leu Trp Ala Asp Ala Gly His Glu Val Ala Trp Glu Gln Phe
                725                 730                 735

Leu Leu Pro Thr Pro Gln Phe Met Ala Gly Gln Asp Gln Asp Ser Val
            740                 745                 750

Leu Thr Ser Asp Arg Gly Val Ile Val Glu Glu Gln Ala Gly Arg Leu
        755                 760                 765

Thr Val Gln Ala Ala Asp Val Ser Leu Gln Phe Ser Thr Ser Ser Gly
    770                 775                 780

Tyr Leu Ile Ser Met Gln Asn Lys Gly Lys Glu Leu Leu Leu Glu Pro
785                 790                 795                 800

Val Arg Pro Asn Phe Trp Arg Ala Val Thr Asp Asn Asp Leu Gly Asn
                805                 810                 815

Lys His His Glu Arg Cys Ala Val Trp Asn Thr Ala Gly Ala Gly Cys
            820                 825                 830

Thr Leu Ala Ser Phe Glu Ser His Lys Asn Val Asp Gly Val Thr Val
        835                 840                 845

Arg Ala Lys Tyr Thr Val Pro Thr Val Pro Ile Ser Ser Leu Ile Leu
    850                 855                 860

Glu Tyr Arg Ile Gln Glu Asn Gly Ser Ile Glu Val Phe Glu Glu Leu
865                 870                 875                 880

Ser Pro Gly Met Gly Leu Pro Glu Ile Pro Glu Ile Gly Leu Met Phe
                885                 890                 895

Ile Val Glu Asp Arg Leu Asp Thr Val Ser Trp Tyr Gly Arg Gly Pro
            900                 905                 910

His Glu Asn Tyr Trp Asp Arg Lys Thr Gly Ala Arg Leu Gly Tyr Phe
        915                 920                 925

Ser Gly Ser Val Gln Asp Gln Phe Val Pro Tyr Ile Arg Pro Gln Glu
    930                 935                 940

Cys Gly Asn Lys Thr Asp Val Arg Phe Ala Ser Ile Thr Gly Gly Ile
945                 950                 955                 960

Asn Gly Ser Gly Phe Arg Val Asp Gly Asp Pro Val Leu Glu Leu Asn
                965                 970                 975

Ala Leu Pro Trp Thr Pro Ala Glu Leu Glu Ala Asn Asp His Ile Tyr
            980                 985                 990

Lys Leu Pro Ala Ser Asn Lys Thr  Val Val Arg Val Asn  Tyr Lys Gln
        995                 1000                 1005

Met Gly  Val Gly Gly Asp Asn  Ser Trp Gly Ala Thr  Thr His Pro
    1010                 1015                 1020

Glu Phe  Thr Leu Pro Ala Asp  Gln Thr Tyr Gly Phe  Arg Phe Thr
    1025                 1030                 1035

Ile Arg  Met Val
    1040

<210>  2
<211>  3129
<212>  DNA
<213>  Paenibacillus wynnii

<400>  2
atgcgtaaaa aacttgtcta ttcaccccct accaacgggt acccggaatg gaataataat      60

cccgaatgtt ttcagatcaa ccgaatggat gcccatgcta catggatacc ttttaataca     120

acagaggatg ccttgcttgg agatccacaa tcctcgccca actatttgtc tttgaatggg     180

atgtggaaat tcgcctatgc agagactccg gatcaacgca tacgtaactt ttttgaaaaa     240

aactatgatt gcagttcttg ggctgaactg aacgttccct ctcattggca gatgcatgga     300

tatgactacc ctcagtatac gaatgtacgg tatccctgga gtgaacgtga gccggagctc     360

aaaccgcctt ttgctcccac ccaatataat ccggttgggt cttatgtacg aaccttctct     420

gtaccggagg actggagcgg gaagcctgtt ttcatcagct ttcaaggtgt ggagtctgcc     480

ttctatgttt ggctcaatgg agagttggtc ggatacagcg aggatacctt cacccctgcg     540

gaatttgact taactcctta tctaatcccg ggagacaata agcttgccgt tgaagtgtat     600

cgttggtgtg acgccagttg gctggaggat caggatttct ggagacttag cggcatattt     660

agagatgtct atctatatac aacaccggaa gcccatattt atgatttctt tgttcgaaca     720

gaactggatg agcagtaccg gcatgctgaa ttgcagctgg atgtgaagct tatggattat     780

tttgaaagaa ccgctgaggc agttattgtg catgcgcagc tttatgatca cgatcagaac 840

gctatctttg atcagccgct ttcacagaca gtctacttca atagcgcttc tacccagacg 900

ctccaattct cttcttctat tattgacccg ttaaaatgga gtgcggaaca tccgaatcta 960

tatacgttag tgttgtcttt acatcatgtg gatggagagc tcatggaagc cgttagctgc 1020

cgtgttggct ttcgtacatt cgagctaaag gacggtttaa tgaagattaa tggaaaacgg 1080

atcgtcttta agggcgtgaa ccgccatgaa ttctcatgcg ataccggcag atcaattgat 1140

gtcgatgata tggtacggga cattctactg atgaaagcac acaacatcaa tgccgttcgg 1200

acttcgcatt atccgaatca gaccatatgg tatgaccttt gtgaccaata tgggctgtat 1260

gtaattgatg agaccaatct ggagacacat ggttcgtgga gctatggaca gacagactta 1320

ggcggaaata cggtacccgg cagtagacct gaatggcgcg cgaatgtact ggatcggtgt 1380

aattcaatgc tccaaaggga caaaaaccac ccttccatcg tcatctggtc actcggtaac 1440

gagtcctttg gcggtgataa ctttgtggct atgcatgatt ttcttaagaa agaagacccc 1500

tcccggcttg tccattatga aggcctcttt cactatcgcg agagcgatgt tgcctcggac 1560

attgagagca ccatgtacat tagtccggcc gatgtggaac agtatgcgct taatgatcca 1620

aagaagcctt atattctatg cgagtatagc catgcaatgg gcaactcctg cgggggattg 1680

catctatact gggaagtttt tgagaaatat gatattttac agggtgcctt tatctgggac 1740

tggattgatc aagcgattcg acttaagcaa gccgacggat ccatgcatat ggcctacggc 1800

ggagacttcg gggaatcccc tcatgacggc aacttctgcg gaaatggctt gattttcgct 1860

gaccgttcag tatctcctaa gctgtatgag gtcaagaaat gctaccagaa tgttaaattt 1920

gaggctgtcg atttagagcg aggcatttat cgggtaacca atcagaatct atttacggat 1980

ttggcggagt acgcactggc ttgggaagta agctgtaacg gcaatcctgt gcttaagggt 2040

actgttgatc tggctgtccc tgcaggagaa tcggcagaaa tttcggttcc cgtggtcgat 2100

gaacctaacc tgcaatccga gggcgagcat gtcctcacgt ttagcctgca attgaagaaa 2160

tccacccttt gggcagacgc aggccatgaa gtggcatggg aacaattcct gctgcctaca 2220

cctcagttta tggcgggtca ggatcaagac tctgttttga catcagatcg tggtgtgatt 2280

gtggaggaac aagcagggcg tttaactgtt caggcggcgg atgtctccct tcaattcagt 2340

acttcaagcg gttatctgat ctctatgcaa aataaaggca aggaactgct gctcgaacct 2400

gttcgtccga atttctggcg tgcagttacg gataacgatc tagggaacaa acaccatgag 2460

cgttgcgcgg tttggaatac agcgggagct ggctgtacac tagcttcctt tgaatcgcat 2520

aagaatgtag atggcgtaac tgttcgtgct aagtacaccg tacccaccgt acccatttct 2580

tcactgatat tggaatatag aatccaagaa aatggctcca ttgaagtctt tgaagagcta 2640

tcaccgggca tgggcttgcc ggagattcct gaaattggcc tcatgtttat cgttgaagat 2700

cgactcgaca ccgtttcatg gtatggccgc ggaccgcatg agaattactg ggaccggaag 2760

acaggcgcca gacttggata tttttcggga agtgtccagg atcagttcgt tccgtatata 2820

aggcctcaag aatgcggaaa caaaacggat gtgcgttttg ccagtatcac tgggggtata 2880

aacggctcag gattccgtgt ggatggcgat cctgtactgg aactaaatgc cttgccttgg 2940

acacccgctg agttggaagc gaatgatcac atatataagc tgccagccag taataaaacc 3000

gttgtgcgtg tgaactacaa gcaaatgggt gtaggtggag acaacagctg gggcgcgacg 3060

acacatcctg aattcacttt gccggcggat caaacttatg ggtttcggtt tacgattcgg 3120

atggtgtaa 3129